# EUROPEAN PATENT SPECIFICATION

(11) **EP 2 605 744 B1**
(45) Date of publication and mention of the grant of the patent: **01.11.2017**
(21) Application number: 10745714.5
(22) Date of filing: 18.08.2010
(51) Int. Cl.: A61K 8/19, A61K 8/44, A61Q 11/00, A61K 8/36

(54) **ORAL CARE PRODUCT AND METHODS OF USE AND MANUFACTURE THEREOF**
MUNDPFLEGEPRODUKTE UND VERFAHREN FÜR DEREN VERWENDUNG UND HERSTELLUNG
PRODUIT DE SOINS ORAUX ET SES PROCÉDÉS D'UTILISATION ET DE FABRICATION

(43) Date of publication of application: 26.06.2013
(73) Proprietor: Colgate-Palmolive Company, New York, NY 10022 (US)
(72) Inventor: ROBINSON, Richard, Scott, Belle Mead NJ 08502 (US); SULLIVAN, Richard, J., Atlantic Highlands NJ 07716 (US); KOHLI, Rajnish, Hillsborough NJ 08844 (US)
(74) Representative: Wibbelmann, Jobst
(86) International application number: PCT/US2010/045894
(87) International publication number: WO 2012/023936

(56) References cited:
- EP-A1- 0 694 298
- EP-A1- 0 711 543
- EP-A2- 0 079 611
- WO-A1-93/19725
- WO-A1-2005/037295
- WO-A2-2009/083643
- CN-A- 101 066 093
- US-A- 6 056 930
- US-A1- 2007 218 111
- US-A1- 2008 131 386
- US-B1- 6 436 370

## Description

### BACKGROUND

Combining basic amino acids with agents having oral care benefits (e.g. fluoride and strontium) to form an oral care product having acceptable long term stability, has proven to be a challenge. In particular, the basic amino acid may raise the pH and facilitate dissociation of strontium ions that can react with fluoride ions to form an insoluble precipitate. Above-neutral pH also has the potential to cause irritation. Neutral or acidic compositions containing arginine bicarbonate may release carbon dioxide, leading to bloating and other undesirable effects. In addition, neutral and acidic conditions increase the potential for formation of an arginine-insoluble calcium complex that has a poorer affinity for the tooth surface. Acidic pH may also reduce any effect the formulation might have on buffering cariogenic lactic acid in the mouth. Finally, less soluble salts, such as calcium carbonate and calcium phosphate, can render the formulations gritty and are less suitable, e.g., for liquid oral care formulations such as mouthwashes.

Because of these formulation hurdles, there remains a need for stable oral care compositions that provide a basic amino acid and efficient delivery of oral care agents such as fluoride and strontium. The compositions of the present invention are directed to that end. Document EP- A- 0 079 611 discloses fluoride containing oral compositions containing strontium EDTA complex.

### SUMMARY

In some embodiments, the present invention provides an oral care composition in the form of a dentifrice or a mouth rinse comprising: an effective amount of a basic amino acid, in free or salt form wherein the basic amino acid is arginine; an effective amount of a water soluble strontium salt selected from strontium acetate, strontium chloride, strontium nitrate, strontium lactate, strontium bromide and mixtures thereof wherein the water-soluble strontium source is present in an amount of 0.1 to 15% by weight; and a fluoride ion source.

In some embodiments, the compositions further comprise a potassium ion source. In some embodiments, the potassium ion source is selected from potassium nitrate and potassium chloride.

In some embodiments, the compositions further comprise additional ingredients selected from: water, an abrasive; a surfactant; a foaming agent; a vitamin; a polymer; an enzyme; a humectant; a thickener; an antimicrobial agent; a preservative, a flavoring agent; a colorant; and a combination of two or more thereof.

In some embodiments, the abrasive is a calcium salt. In some embodiments, the calcium salt has poor water solubility. In some embodiments, the calcium salt is selected from: calcium carbonate; calcium phosphate; and calcium chloride.

In those embodiments where the compositions are in the form of a dentifrice, the dentifrice comprises from about 0.1 to about 15%, by weight, of a water soluble strontium salt. In some embodiments, the dentifrice may comprise from about 8 to about 10%, by weight, of a water soluble strontium salt.

In those embodiments where the compositions are in the form of a mouth rinse, the mouth rinse comprises from about 0.01 to about 2%, by weight, of a water soluble strontium salt. In some embodiments, the mouth rinse may comprise from about 0.1 to about 1%, by weight, of a water soluble strontium salt.

Without intending to be bound by a particular theory, it is hypothesized that a significant factor in the beneficial effect of arginine is that arginine and other basic amino acids can be metabolized by certain types of bacteria, e.g., *S. sanguis* which are not cariogenic and which compete with cariogenic bacteria such as *S*. *mutans,* for position on the teeth and in the oral cavity. The arginolytic bacteria can use arginine and other basic amino acids to produce ammonia, thereby raising the pH of their environment, while cariogenic bacteria metabolize sugar to produce lactic acid, which tends to lower the plaque pH and demineralize the teeth, ultimately leading to cavities. It is believed that regular use of a composition, over time, will lead to a relative increase in the arginolytic bacteria and a relative decrease in the cariogenic bacteria, resulting in a higher plaque pH, in effect immunizing the teeth against cariogenic bacteria and their detrimental effects. It is believed that this pH-raising effect may be mechanistically separate from and complementary to the effect of fluoride in promoting remineralization and strengthening the tooth enamel.

Irrespective of the precise mechanism, however, it is surprisingly found that the combination of strontium and a basic amino acid, e.g., arginine, in an oral care product produces unexpected benefits beyond and qualitatively different from what can be observed using compositions comprising effective amounts of each of the compounds separately, in promoting remineralization, repairing pre-carious lesions, and enhancing oral health. It has moreover been found that this action can be further enhanced by addition of a small particle abrasive, which may act to help fill microfissures in the enamel and microtubules in the dentin.

The presence of a basic amino acid in combination with an anionic surfactant is also surprisingly found to reduce bacterial adhesion to the tooth surface. The basic amino acid together with an anionic surfactant also substantially enhances solubilization, release, delivery, deposition, and effectiveness of poorly soluble active agents, for example antimicrobial agents, such as triclosan.

Some embodiments of the present invention provide compositions defined herein for use in methods to: (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) reduce levels of acid producing bacteria, (vii) to increase relative levels of arginolytic bacteria, (viii) inhibit microbial biofilm formation in the oral cavity, (ix) raise and/or maintain plaque pH at levels of at least pH 5.5 following sugar challenge, (x) reduce plaque accumulation, and/or (xi) reduce teeth erosion, comprising applying any one of the compositions described herein to a surface of the oral cavity of a subject in need thereof.

### DETAILED DESCRIPTION

As used throughout, ranges are used as shorthand for describing each and every value that is within the range. Any value within the range can be selected as the terminus of the range.

In certain embodiments, an oral care composition in the form of a dentifrice or a mouth rinse (Composition 1.0) comprises:
an effective amount of a basic amino acid, in free or salt form wherein the basic amino acid is arginine; an effective amount of a water soluble strontium salt, wherein the water-soluble strontium salt is selected from strontium acetate; strontium chloride;
strontium nitrate; strontium lactate; strontium bromide; and a combination of two or more thereof; wherein the water soluble strontium salt is present in an amount of from 0.1 to 15% by weight; and a fluoride ion source.

In some embodiments, the composition further comprises a potassium ion source. In some embodiments, the potassium ion source is selected from potassium nitrate and potassium chloride.

For example, any of the following compositions:
1.0.1.
1.0.2. Composition 1.0 wherein the arginine acid has the L-configuration.
1.0.3. Any of the preceding compositions provided in the form of a di- or tri-peptide comprising the basic amino acid, or salts thereof.
1.0.4.
1.0.5.
1.0.6. Any of the preceding compositions wherein the basic amino acid is partially or wholly in salt form.
1.0.7. Composition 1.0.6 wherein the basic amino acid is arginine phosphate.
1.0.8. Composition 1.0.6 wherein the basic amino acid is arginine hydrochloride.
1.0.9. Composition 1.0.6 wherein the basic amino acid is arginine bicarbonate.
1.0.10. Any of the preceding compositions wherein a salt of the basic amino acid is formed in situ in the formulation by neutralization of the basic amino acid with an acid or a salt of an acid.
1.0.11. Any of the preceding compositions wherein the salt of the basic amino acid is formed by neutralization of the basic amino acid to form a premix prior to combination with a fluoride salt.
1.0.12. Any of the preceding compositions wherein the basic amino acid is present in an amount from about 0.1 to about 20%, by weight. In some embodiments, the basic acid is present in an amount of about 1 to about 10 %, by weight, of the composition, the weight of the basic amino acid being calculated as free base form.
1.0.13. Composition 1.0.11 wherein the basic amino acid is present in an amount of about 7.5%, by weight, of the composition.
1.0.14. Composition 1.0.11 wherein the basic amino acid is present in an amount of about 5 %, by weight, of the composition.
1.0.15. Composition 1.0.11 wherein the basic amino acid is present in an amount of about 3.75%, by weight, of the composition.
1.0.16. Composition 1.0.11 wherein the basic amino acid is present in an amount of about 1.5%, by weight, of the composition.
1.0.17. Any of the foregoing compositions wherein the water soluble strontium salt is selected from strontium acetate, strontium chloride, strontium nitrate, strontium lactate and strontium bromide; and mixtures thereof.
1.0.18. Any of the foregoing compositions wherein the water soluble strontium salt is strontium acetate.
1.0.19. Any of the preceding compositions comprising a fluoride ion source. In some embodiments, the fluoride ion source is selected from sodium monoflurophosphate, stannous fluoride, sodium fluoride, potassium fluoride, sodium fluorosilicate, ammonium fluorosilicate, amine fluoride, ammonium fluoride, and a combination of two or more thereof. Some embodiments provide a fluoride ion source wherein a fluoride ion is covalently bond to another atom.
1.0.20. Any of the preceding compositions wherein the fluoride ion source is a fluorophosphate.
1.0.21. Any of the preceding composition wherein the fluoride ion source is sodium monofluorophosphate.
1.0.22. Any of the preceding compositions wherein the fluoride ion source is present in an amount of about 0.01 to about 2 %, by weight, of the composition.
1.0.23. Any of the preceding compositions wherein the fluoride ion source provides fluoride ion in an amount of about 0.1 to about 0.2%, by weight, of the composition.
1.0.24. Any of the preceding compositions wherein the fluoride ion source provides fluoride in an amount of from about 50 to about 25,000 ppm.
1.0.25. Any of the preceding compositions which is a mouthwash, wherein the fluoride ion source provides fluoride in an amount of from about 100 to about 250 ppm..
1.0.26. Any of the preceding compositions which is a dentifrice, wherein the fluoride ion source provides fluoride in an amount of from about 750 to about 2000 ppm..
1.0.27. Any of the preceding compositions wherein the fluoride ion source provides fluoride in an amount of from about 1000 to about 1500 ppm.
1.0.28. Any of the preceding compositions wherein the fluoride ion source provides fluoride in the amount of 1450 ppm..
1.0.29. Any of the preceding compositions wherein the pH is 6 to 9, e.g., 6.5 to 7.4 or 7.5 to 9.
1.0.30. Any of the preceding compositions wherein the pH is 6.5 to 7.4.
1.0.31. Any of the preceding compositions wherein the pH is 6.8 to 7.2.
1.0.32. Any of the preceding compositions wherein the pH is approximately neutral.
1.0.33. Any of the preceding compositions further comprising an anti-calculus agent.
1.0.34. Any of the preceding compositions further comprising an anti-calculus agent which is a polyphosphate, e.g., pyrophosphate, tripolyphosphate, or hexametaphosphate, e.g., in sodium salt form.
1.0.35. Any of the preceding compositions further comprising an abrasive or particulate.
1.0.36. The immediately preceding composition wherein the abrasive or particulate is selected from sodium bicarbonate, calcium phosphate (e.g.,dicalcium phosphate dihydrate), calcium sulfate, precipitated calcium carbonate, silica (e.g. hydrated silica), iron oxide, aluminium oxide, perlite, plastic particles, e.g., polyethylene, and combinations thereof.
1.0.37. The immediately preceding composition wherein the abrasive or particulate is selected from a calcium phosphate (e.g., dicalcium phosphate dihydrate), calcium sulfate, precipitated calcium carbonate, silica (e.g. hydrated silica), and a combination of two or more thereof.
1.0.38. Any of the preceding compositions comprising an abrasive in an amount of about 15 to about 70%, by weight, of the composition.
1.0.39. Any of the preceding compositions comprising a small particle abrasive fraction of at least 5% having a d50 of < 5 micrometers.
1.0.40. Any of the preceding compositions having a RDA of less than 150, e.g., 40 to 140.
1.0.41. Any of the preceding compositions wherein the anionic surfactant is selected from
   a. water-soluble salts of higher fatty acid monoglyceride monosulfates (e.g. the sodium salt of the monosulfated monoglyceride of hydrogenated coconut oil fatty acids such as sodium N-methyl N-cocoyl taurate, sodium cocomo-glyceride sulfate),
   b. higher alkyl sulfates, e.g. sodium lauryl sulfate,
   c. higher alkyl-ether sulfates, e.g., of formula CH₃(CH₂)ₘCH₂(OCH₂CH₂)ₙOSO₃X, wherein m is 6-16, e.g. 10, n is 1-6, e.g., 2, 3 or 4, and X is Na or K (for example sodium laureth-2 sulfate (CH₃(CH₂)₁₀CH₂(OCH₂CH₂)₂OSO₃Na)),
   d. higher alkyl aryl sulfonates (such as sodium dodecyl benzene sulfonate (sodium lauryl benzene sulfonate)),
   e. higher alkyl sulfoacetates (such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate),
   f. and mixtures thereof.
   By "higher alkyl" is meant, e.g. C₆₋₃₀ alkyl. In particular embodiments, the anionic surfactant is selected from sodium lauryl sulfate and sodium ether lauryl sulfate.
1.0.42. Any of the preceding compositions wherein the anionic surfactant is selected from sodium lauryl sulfate, sodium ether lauryl sulfate, and mixtures thereof.
1.0.43. Any of the preceding compositions wherein the anionic surfactant is present in an amount of about 0.3 to about 4.5% by weight.
1.0.44. Any of the preceding compositions further comprising a surfactant selected from a cationic, zwitterionic, nonionic surfactant; and a mixture of two or more thereof.
1.0.45. Any of the preceding compositions comprising at least one humectant.
1.0.46. Any of the preceding compositions comprising at least one humectant selected from glycerin, sorbitol and combinations thereof.
1.0.47. Any of the preceding compositions comprising xylitol.
1.0.48. Any of the preceding compositions comprising at least one polymer.
1.0.49. Any of the preceding compositions comprising at least one polymer selected from polyethylene glycols, polyvinylmethyl ether maleic acid copolymers, polysaccharides (e.g. cellulose derivatives, for example carboxymethyl cellulose, or polysaccharide gums, for example xanthan gum or carrageenan gum), and combinations thereof.
1.0.50. Any of the preceding compositions comprising gum strips or fragments.
1.0.51. Any of the preceding compositions comprising a flavoring agent, fragrance and/or colorant.
1.0.52. Any of the preceding compositions comprising water.
1.0.53. Any of the preceding compositions comprising an antibacterial agent selected from triclosan, herbal extracts and essential oils (e.g. rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract, propolis), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.0.54. Any of the preceding compositions comprising an anti-inflammatory compound, e.g., an inhibitor of at least one of host pro-inflammatory factors selected from matrix metalloproteinases (MMP's), cyclooxygenases (COX), PGE₂, interleukin 1 (IL-1), IL-1β converting enzyme (ICE), transforming growth factor β1 (TGF-β1), inducible nitric oxide synthase (iNOS), hyaluronidase, cathepsins, nuclear factor kappa B (NF-κB), and IL-1 Receptor Associated Kinase (IRAK), e,g, selected from aspirin, ketorolac, flurbiprofen, ibuprofen, naproxen, indomethacin, aspirin, ketoprofen, piroxicam, meclofenamic acid, nordihydoguaiaretic acid, and mixtures thereof.
1.0.55. Any of the preceding compositions comprising an antioxidant, e.g., selected from the group consisting of Co-enzyme Q10, PQQ, Vitamin C, Vitamin E, Vitamin A, anethole-dithiothione, and mixtures thereof.
1.0.56. Any of the preceding compositions wherein the anti-microbial is poorly soluble in water.
1.0.57. Any of the preceding compositions comprising triclosan.
1.0.58. Any of the preceding compositions comprising triclosan and xylitol.
1.0.59. Any of the preceding compositions comprising triclosan, xylitol, and precipitated calcium carbonate.
1.0.60. Any of the preceding compositions comprising triclosan and a zinc ion source, e.g., zinc citrate.
1.0.61. Any of the preceding compositions comprising an antibacterial agent in an amount of about 0.01 to about 5%, by weight, of the composition.
1.0.62. Any of the preceding compositions comprising triclosan in an amount of about 0.01 to about 1%, by weight, of the composition.
1.0.63. Any of the preceding compositions comprising triclosan in an amount of 0.3%, by weight, of the composition.
1.0.64. Any of the preceding compositions, further comprising a whitening agent.
1.0.65. Any of the preceding compositions, further comprising a whitening agent selected from the group consisting of a peroxide, a metal chlorite, a perborate, a percarbonate, a peroxyacid, a hypochlorite, and a combination of two or more thereof.
1.0.66. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.
1.0.67. Any of the preceding compositions further comprising an agent that interferes with or prevents bacterial attachment, e.g. solbrol or chitosan.
1.0.68. Any of the preceding compositions further comprising a source of strontium and phosphate selected from (i) strontium-glass complexes, e.g., strontium sodium phosphosilicates, and (ii) strontium-protein complexes, e.g., casein phosphopeptide-amorphous strontium phosphate.
1.0.69. Any of the preceding compositions further comprising a potassium ion source, e.g., potassium nitrate or potassium chloride, in an amount effective to reduce dentinal sensitivity.
1.0.70. Any of the preceding compositions comprising about 0.1 to about 7.5%, by weight, of a potassium ion source, e.g., potassium nitrate and/or potassium chloride.
1.0.71. Any of the preceding compositions effective upon application to the oral cavity, e.g., with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) reduce dry mouth, (xiii) clean the teeth and oral cavity (xiv) reduce erosion, (xv) whiten teeth, and/or (xvi) immunize the teeth against cariogenic bacteria.
1.0.72. A composition obtained or obtainable by combining the ingredients as set forth in any of the preceding compositions.
1.0.73. Any of the preceding compositions in the form of a dentifrice, e.g. in a form selected from toothpaste, tooth gel, tooth powder, non-abrasive gel, mousse, foam, mouth spray, lozenge and oral tablet.
1.0.74. Any of the preceding compositions wherein the composition is toothpaste.
1.0.75. Any of the preceding compositions wherein the composition is a toothpaste optionally further comprising one or more of one or more of water, an abrasive, a surfactant, a foaming agent, a vitamin, a polymer, an enzyme, a humectant, a thickener, an antimicrobial agent, a preservative, a flavoring agent, a colorant and/or combinations thereof.
1.0.76. Any of the preceding compositions wherein the composition is a mouthrinse.
1.0.77. Any of the preceding compositions further comprising a breath freshener, fragrance or flavoring agent.

Levels of active ingredients will vary based on the nature of the delivery system and the particular active. For example, the basic amino acid may be present at levels from, e.g. about 0.1 to about 20%, by weight, of the composition (expressed as weight of free base), e.g. about 0.1 to about 3%, by weight, for a mouth rinse, about 1 to about 10%, by weight, for a consumer toothpaste or about 7 to about 20%, by weight, for a professional or prescription treatment product. The fluoride ion source may provide fluoride at levels of, e.g. 25 to 25,000 ppm, for example 25 to 250 ppm for a mouth rinse, 750 to 2,000 ppm for a consumer toothpaste, or 2,000 to 25,000 ppm for a professional or prescription treatment product. Levels of antibacterial will vary similarly, with levels used in toothpaste being e.g. 5 to 15 times greater than used in mouth rinse. For example, a triclosan mouth rinse may contain, e.g. 0.03%, by weight, triclosan; while a triclosan toothpaste may contain 0.3%, by weight, triclosan.

The water soluble strontium salts are present in an amount of from about 0.1 to about 15%, by weight, e.g. about 0.1 to about 2%, by weight, for a mouth rinse; and about 6 to about 10%, by weight, or higher for a dentifrice.

In other embodiments, the invention provides a composition as defined herein for use in a method to improve oral health comprising applying an effective amount of the oral composition to the oral cavity of a subject in need thereof, e.g. a method to:
i. reduce or inhibit formation of dental caries,
ii. reduce, repair or inhibit early enamel lesions, e.g., as detected by quantitative light-induced fluorescence (QLF) or electrical caries measurement (ECM),
iii. reduce or inhibit demineralization and promote remineralization of the teeth,
iv. reduce hypersensitivity of the teeth,
v. reduce or inhibit gingivitis,
vi. reduce levels of acid producing bacteria,
vii. increase relative levels of arginolytic bacteria,
viii. inhibit microbial biofilm formation in the oral cavity,
ix. raise and/or maintain plaque pH at levels of at least pH 5.5 following sugar challenge,
x. reduce plaque accumulation,
xi. reduce erosion of the teeth, and/or
xii. clean the teeth and oral cavity.

Other embodiments provide the use of arginine in the manufacture of a composition described above for use in any of the indications set forth in the above method.

### Basic Amino Acids

In certain embodiments, the basic amino acid is L-arginine, or a salt thereof.

The compositions are intended for topical use in the mouth and so salts for use in the composition should be safe for such use, in the amounts and concentrations provided. Suitable salts include salts known in the art to be pharmaceutically acceptable. Salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as magnesium. Physiologically acceptable salts may be obtained using standard procedures known in the art, for example, by reacting a sufficiently basic compound such as an amine with a suitable acid affording a physiologically acceptable anion.

In various embodiments, the basic amino acid is present in an amount of from about 0.5 to about 20%, by weight, of the composition. In some embodiments, the basic amino acid is present in an amount of from about 1 to about 10%, by weight, of the composition; for example about 1.5 %, about 3.75%, about 5%, or about 7.5%, by weight, of the composition.

### RDA

RDA is an abbreviation for radioactive dentin abrasion, a relative measure of abrasivity. Typically, extracted human or cow teeth are irradiated in a neutron flux, mounted in methylmethacrylate (bone glue), stripped of enamel, inserted into a brushing-machine, brushed by American Dental Association (ADA) standards (reference toothbrush, 150g pressure, 1500 strokes, 4-to-1 water-toothpaste slurry). The radioactivity of the rinsewater is then measured and recorded. For experimental control, the test is repeated with an ADA reference toothpaste made of calcium pyrophosphate, with this measurement given a value of 100 to calibrate the relative scale.

In the invention, the strontium salt is selected from strontium acetate, strontium chloride, strontium nitrate, strontium lactate, strontium bromide and mixtures thereof. In some embodiments, the strontium salt is strontium acetate. In some embodiments, the strontium acetate is in the form of its hemihydrate. In some embodiments, the strontium acetate is present in the composition in the amount of about 1 to about 10% by weight. In some embodiments, this content in the composition refers to the weight of the component in its hemihydrate form. In some embodiments, the strontium acetate hemihydrate is present in the composition in an amount of from about 5 to about 8%, by weight.

### Fluoride Ion Sources

The oral care compositions of the invention comprise a fluoride ion source, e.g., soluble fluoride salts. As free fluoride ions may react in aqueous solution with free calcium or strontium ions, the fluoride may be covalently bound to another atom, e.g., selected from fluorophosphates e.g., sodium monofluorophosphate, fluorosilicates, e.g., sodium fluorosilicate, ammonium fluorosilicate, and fluorosulfates, e.g., hexafluorosulfate, and combinations thereof; or the fluoride may be sequestered from the calcium or strontium ions and/or either the fluoride or the strontium or both provided in a nonaqueous system.

A wide variety of fluoride ion sources can be employed as sources of water soluble fluoride in the present compositions. Examples of suitable fluoride ion sources are described in U.S. Pat. No. 3,535,421, to Briner et al.; U.S. Pat. No. 4,885,155, to Parran, Jr. et al. and U.S. Pat. No. 3,678,154, to Widder et al.

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. As noted, because of the potential for reaction with the strontium in the compositions, however, where the fluoride ion source is provided in solution with the compositions, they are preferably salts wherein the fluoride is covalently bound to another atom, e.g., as in sodium monofluorophosphate, rather than merely ionically bound, e.g., as in sodium fluoride.

In certain embodiments, the oral care composition comprises a fluoride ion source in an amount sufficient to supply 25 ppm to 25,000 ppm of fluoride ions, generally at least 500 ppm, e.g., 500 to 2000 ppm, e.g., 1000 to 1600 ppm, e.g., 1450 ppm. The appropriate level of fluoride will depend on the particular application. A mouthwash, for example, would typically have 100 to 250 ppm fluoride. A toothpaste for general consumer use would typically have 1000 1500 ppm, with pediatric toothpaste having somewhat less. A dentifrice or coating for professional application could have as much as 5,000 or even 25,000 ppm fluoride.

Fluoride ion sources may be added to the compositions in an amount of about 0.01 to about 10%, by weight, from about 0.03 to 5 %, by weight, or from about 0.1 % to about 1%, by weight, of the composition. The weight of fluoride ion source which provides the appropriate level of fluoride ion will obviously vary based on the weight of the counter ion in the salt.

### Abrasives

The compositions may comprise a calcium phosphate abrasive, e.g., tricalcium phosphate (Ca₃(PO₄)₂), hydroxylapatite (Ca₁₀(PO₄)₆(OH)₂), or dicalcium phosphate dihydrate (CaHPO₄ • 2H₂O, also sometimes referred to herein as DiCal); or other poorly water soluble calcium salt, e.g., calcium carbonate.

The compositions may include one or more additional abrasives, for example silica abrasives such as precipitated silicas having a mean particle size of up to 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof.

The silica abrasive polishing materials useful herein, as well as the other abrasives, generally have an average particle size of 0.1 and 30 microns, 5 to 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason.

In certain embodiments, abrasive materials useful in the practice of the oral care compositions include silica gels and precipitated amorphous silica having an oil absorption value of less than 100 cc/100 g silica and in the range of 45 cc/100 g to 70 cc/100 g silica. Oil absorption values are measured using the ASTA Rub-Out Method D281. In certain embodiments, the silicas are colloidal particles having an average particle size of 3 microns to 12 microns, and 5 to 10 microns.

In particular embodiments, the abrasive materials comprise very small particles, e.g. having a d50 < 5 microns, for example, small particle silica (SPS) having a d50 of 3 to 4 microns, for example Sorbosil AC43® (PQ Corporation). Such small particles are particularly useful in formulations targeted at reducing hypersensitivity. The small particle component may be present in combination with a second larger particle abrasive. In certain embodiments, for example, the formulation comprises 3 to 8% small particles e.g., SPS and 25 to 45% of a conventional abrasive.

Low oil absorption silica abrasives that are particularly useful in certain embodiments are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of about 29% by weight averaging 7 to 10 microns in diameter, and an oil absorption of less than 70 cc/100 g of silica is an example of a low oil absorption silica abrasive useful in the composition. The abrasive is present in the oral care composition at a concentration of 10 to 60% by weight, in other embodiment 20 to 45% by weight, and in another embodiment 30 to 50% by weight.

### Foaming Agents

The oral care compositions also may include an agent to increase the amount of foam that is produced when the oral cavity is brushed.

Illustrative examples of agents that increase the amount of foam include, but are not limited to polyoxyethylene and certain polymers including, but not limited to, alginate polymers.

The polyoxyethylene may increase the amount of foam and the thickness of the foam generated by the oral care carrier component. Polyoxyethylene is also commonly known as polyethylene glycol ("PEG") or polyethylene oxide. The polyoxyethylenes suitable in certain embodiments will have a molecular weight of 200,000 to 7,000,000. In one embodiment the molecular weight will be 600,000 to 2,000,000 and in another embodiment 800,000 to 1,000,000. Polyox® is the trade name for the high molecular weight polyoxyethylene produced by Union Carbide.

The polyoxyethylene may be present in an amount of 1% to 90%, in one embodiment 5% to 50% and in another embodiment 10% to 20% by weight of the oral care carrier component of the oral care composition. The dosage of foaming agent in the oral care composition (i.e., a single dose) is 0.01 to 0.9 % by weight, 0.05 to 0.5% by weight, and in another embodiment 0.1 to 0.2 % by weight.

### Surfactants

In certain embodiments, the composition comprises an anionic surfactant, for example
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomo-glyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g. 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.
By "higher alkyl" is meant, e.g. C₆₋₃₀ alkyl. In particular embodiments, the anionic surfactant is selected from sodium lauryl sulfate and sodium ether lauryl sulfate.

In some embodiments, the anionic surfactant is present in an amount which is effective, e.g., > 0.01% by weight of the formulation, but not at a concentration which would be irritating to the oral tissue, e.g., < 10%, and optimal concentrations depend on the particular formulation and the particular surfactant. For example, concentrations used or a mouthwash are typically on the order of one tenth that used for a toothpaste. In some embodiments, the anionic surfactant is present in a toothpaste at from about 0.3 to about 4.5%, by weight, e.g., 1.5%.

The composition may optionally contain mixtures of surfactants, comprising anionic surfactants and other surfactants which may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al.

In certain embodiments, the anionic surfactants useful herein include the water-soluble salts of alkyl sulfates having 10 to 18 carbon atoms in the alkyl radical and the water-soluble salts of sulfonated monoglycerides of fatty acids having 10 to 18 carbon atoms. Sodium lauryl sulfate, sodium lauroyl sarcosinate and sodium coconut monoglyceride sulfonates are examples of anionic surfactants of this type. Mixtures of anionic surfactants may also be utilized.

In other embodiments, cationic surfactants can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

Illustrative cationic surfactants are the quaternary ammonium fluorides described in U.S. Pat. No. 3,535,421, to Briner et al. Certain cationic surfactants can also act as germicides in the compositions.

In certain embodiments, nonionic surfactants that can be used in the compositions can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants can be broadly described as derivatives of aliphatic quaternary ammonium, phosphomium, and sulfonium compounds, in which the aliphatic radicals can be straight chain or branched, and wherein one of the aliphatic substituents contains 8 to 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

In a particular embodiment, the composition comprises sodium lauryl sulfate.

The surfactant or mixtures of compatible surfactants can be present in the composition at about 0.1 to about 5%, by weight; in another embodiment at about 0.3 to about 3%, by weight; and in another embodiment at about 0.5 to about 2%, by weight, of the composition.

### Flavoring Agents

The oral care composition may also include a flavoring agent. Examples of flavoring agents include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

In some embodiments, the flavoring agent comprises from about 0.1 to about 5%, by weight, of the composition. In other embodiments, the flavoring agent comprises from about 0.5 to about 1.5%, by weight, of the composition. In some embodiments, the dosage of flavoring agent in the individual oral care composition dosage (i.e., a single dose) is 0.001 to 0.05% by weight, while in other embodiments the dosage of flavoring agent is 0.005 to 0.015 % by weight.

### Chelating agents

The oral care composition also may optionally include one or more chelating agents able to complex calcium found in the cell walls of the bacteria. Binding of this calcium weakens the bacterial cell wall and augments bacterial lysis.

Another group of agents suitable for use as chelating agents are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 1%, by weight, pyrophosphate ions. In some embodiments, the pyrophosphate salt provides 1.5%, by weight, pyrophosphate ions. In some embodiments, the pyrophosphate salt provides 6%, by weight, pyrophosphate ions. In some embodiments, the pyrophosphate salt provides from about 3.5 to about 6%, by weight, pyrophosphate ions.

### Polymers

The oral care compositions also optionally include one or more polymers, such as polyethylene glycols, polyvinylmethyl ether maleic acid copolymers, polysaccharides (e.g. cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g. potassium and sodium) or ammonium salts.

Particularly when noncationic antibacterial agents or antibacterial agents, e.g., triclosan, are included in any of the dentifrice components, there is also preferably included from 0.05 to 5% of an agent which enhances the delivery and retention of the agents to, and retention thereof on oral surfaces. Non limiting examples of such agents are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of 30,000 to 1,000,000, most preferably 30,000 to 800,000. These copolymers are available for example as Gantrez. e.g. AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts of 0.05 to 3% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of 1,000 to 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al.

### Thickening Agents

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of 0.5% to 5% by weight of the total composition are used.

### Enzymes

The oral care composition may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes that can be used are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191. In some embodiments, the composition comprises from about 0.002 to about 2%, by weight, of an enzyme. In some embodiments, the composition comprises from about 0.05 to about 1.5%, by weight, of an enzyme. In other embodiments, the composition comprises from about 0.1 to about 0.5%, by weight, of an enzyme.

### Water

Water may also be present in the oral composition. Water, employed in the preparation of commercial oral compositions should be deionized and free of organic impurities. Water commonly makes up the balance of the compositions and includes 10% to 90%, 20% to 60% or 10% to 30% by weight of the oral compositions. This amount of water includes the free water which is added plus that amount which is introduced with other materials such as with sorbitol or any other component.

### Humectants

Within certain embodiments of the invention, it is also desirable to incorporate a humectant to prevent the composition from hardening upon exposure to air. Certain humectants can also impart desirable sweetness or flavor to dentifrice compositions. The humectant, on a pure humectant basis, may be included in an amount of from about 15 to about 70%, by weight, in some embodiments; and from about 30 to about 65%, by weight, in other embodiments.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, other embodiments can contain a variety of optional dentifrice ingredients some of which are described herein. Optional ingredients include, for example, but are not limited to, adhesives, sudsing agents, flavoring agents, sweetening agents, additional antiplaque agents, abrasives, and colorants. These and other optional components are further described in U.S. Pat. No. 5,004,597, to Majeti; U.S. Pat. No. 3,959,458 to Agricola et al. and U.S. Pat. No. 3,937,807, to Haefele.

### Methods of Manufacture

The compositions can be made using methods which are common in the oral product area.

In one illustrative embodiment, the oral care composition is made by neutralizing arginine in a gel phase with phosphoric acid and mixing to form Premix 1.

Actives such as, for example, a strontium salt, vitamins, cetyl pyridinium chloride, a fluoride ion source, an abrasive, and any other desired active ingredients are added to Premix 1 and mixed to form Premix 2.

A toothpaste base, for example, dicalcium phosphate is added to Premix 2 and mixed. The final slurry is formed into an oral care product.

### Methods

Some embodiments of the present invention provide compositions as defined herein for use in methods to protect the teeth by facilitating repair and remineralization, in particular to reduce or inhibit formation of dental caries, reduce or inhibit demineralization and promote remineralization of the teeth, reduce hypersensitivity of the teeth, and reduce, repair or inhibit early enamel lesions, e.g., as detected by quantitative light-induced fluorescence (QLF) or electronic caries monitor (ECM); comprising applying to the oral cavity a safe and effective amount of any one of the compositions described herein.

Quantitative Light-induced Fluorescence is a visible light fluorescence that can detect early lesions and longitudinally monitor the progression or regression. Normal teeth fluoresce in visible light; demineralized teeth do not or do so only to a lesser degree. The area of demineralization can be quantified and its progress monitored. Blue laser light is used to make the teeth auto fluoresce. Areas that have lost mineral have lower fluorescence and appear darker in comparison to a sound tooth surface. Software is used to quantify the fluorescence from a white spot or the area/volume associated with the lesion. Generally, subjects with existing white spot lesions are recruited as panelists. The measurements are performed in vivo with real teeth. The lesion area/volume is measured at the beginning of the clinical. The reduction (improvement) in lesion area/volume is measured at the end of 6 months of product use. The data is often reported as a percent improvement versus baseline.

Electrical Caries Monitoring is a technique used to measure mineral content of the tooth based on electrical resistance. Electrical conductance measurement exploits the fact that the fluid-filled tubules exposed upon demineralization and erosion of the enamel conduct electricity. As a tooth loses mineral, it becomes less resistive to electrical current due to increased porosity. An increase in the conductance of the patient's teeth therefore may indicate demineralization. Generally, studies are conducted of root surfaces with an existing lesion. The measurements are performed in vivo with real teeth. Changes in electrical resistance before and after 6 month treatments are made. In addition, a classical caries score for root surfaces is made using a tactile probe. The hardness is classified on a three point scale: hard, leathery, or soft. In this type of study, typically the results are reported as electrical resistance (higher number is better) for the ECM measurements and an improvement in hardness of the lesion based on the tactile probe score.

The oral care compositions are thus useful in a method to reduce early lesions of the enamel (as measured by QLF or ECM) relative to a composition lacking effective amounts of fluorine and/or arginine.

In some embodiments, the oral care compositions can be used to reduce harmful bacteria in the oral cavity. In some embodiments, the oral care compositions can be used to reduce or inhibit gingivitis. In some embodiments, the oral care compositions can be used to reduce levels of acid producing bacteria. In some embodiments, the oral care compositions can be used to increase relative levels of arginolytic bacteria. In some embodiments, the oral care compositions can be used to inhibit microbial biofilm formation in the oral cavity. In some embodiments, the oral care compositions can be used to raise and/or maintain plaque pH at levels of at least pH about 5.5 following sugar challenge. In some embodiments, the oral care compositions can be used to reduce plaque accumulation. In some embodiments, the oral care compositions can be used to clean the teeth and oral cavity.

Finally, by increasing the pH in the mouth and discouraging pathogenic bacteria, the oral care compositions are useful to promote healing of sores or cuts in the mouth.

The compositions and methods can be incorporated into oral compositions for the care of the mouth and teeth such as toothpastes, transparent pastes, gels, mouth rinses, sprays and chewing gum. The invention specifically provides a dentifrice or a mouth rinse.

Enhancing oral health also provides benefits in promoting systemic health, as the oral tissues can be gateways for systemic infections. Good oral health is associated with systemic health, including cardiovascular health. The compositions and methods provide particular benefits because basic amino acids, especially arginine, are sources of nitrogen which supply NO synthesis pathways and thus enhance microcirculation in the oral tissues. Providing a less acidic oral environment is also helpful in reducing gastric distress and creates an environment less favorable to Heliobacter, which is associated with gastric ulcers. Arginine in particular is required for high expression of specific immune cell receptors, for example T-cell receptors, so that arginine can enhance an effective immune response. The compositions and methods are thus useful to promote systemic health, including cardiovascular health.

The following examples further describe and demonstrate illustrative embodiments within the scope of the present invention.

### EXAMPLES

### Example 1 (Outside the scope of the invention)

A compsition comprising the ingredients described in Table 1 (below) can be prepared by any of the methods described above.

**Table 1**

| **RAW MATERIAL** | **WEIGHT %** |
|---|---|
| Xylitol | 2 |
| L-Arginine | 0.5 |
| Hydroxyethyl cellulose | 0.43 |
| Dibasic potassium phosphate | 0.08 |
| Potassium chloride | 0.062 |
| Potassium phosphate monobasic | 0.043 |
| Strontium Acetate | 0.01 |
| Magnesium chloride | 0.0059 |
| Sodium fluoride | 0.00045 |
| Deionized Water, colorant, preservative, and flavoring agent(s) | qs |
| **TOTAL** | 100 |

### Example 2

A compsition comprising the ingredients described in Table 2 (below) can be prepared by any of the methods described above.

**Table 2**

| **RAW MATERIAL** | **WEIGHT %** |
|---|---|
| Glycerin | 10 |
| 70% Sorbitol | 10 |
| 95% Ethanol | 6 |
| Polysorbate 20 | 1 |
| Sodium benzoate | 0.11 |
| Strontium Acetate | 0.6 |
| Phosphoric acid 85% | 0.08 |
| L-Arginine | 0.6 |
| Deionized Water, flavoring agent(s), sweeteners, colorant | qs |
| TOTAL | 100.000 |
| **pH** | **9.0** |

## Claims

1. An oral care composition in the form of a dentifrice or a mouth rinse, the composition comprising
a. an effective amount of a basic amino acid, in free or salt form, wherein the basic amino acid is arginine;
b. an effective amount of a water soluble strontium salt, wherein the water soluble strontium salt is selected from strontium acetate; strontium chloride; strontium nitrate; strontium lactate; strontium bromide; and a combination of two or more thereof; wherein the water soluble strontium salt is present in an amount of from 0.1 to 15% by weight,
wherein the composition further comprises a fluoride ion source.

2. The oral care composition according to claim 1 wherein the fluoride ion source is selected from sodium monofluorophosphate; stannous fluoride; sodium fluoride; potassium fluoride; sodium fluorosilicate; ammonium fluorosilicate; amine fluoride; ammonium fluoride; and a combination of two or more thereof.

3. The oral care composition of claim 1 or claim 2, further comprising a potassium ion source.

4. The oral care composition of claim 3, wherein said potassium ion source is selected from potassium nitrate and potassium chloride.

5. The oral care composition of any preceding claim, further comprising a strontium-glass complex.

6. The oral care composition of claim 5, wherein the strontium-glass complex comprises strontium sodium phosphosilicate.

7. An oral care composition according to any one of claims 1 to 6, for use in a method of
a. reducing or inhibiting formation of dental caries,
b. reducing, repairing or inhibiting early enamel lesions,
c. reducing or inhibiting demineralization and promoting remineralization of the teeth,
d. reducing hypersensitivity of the teeth,
e. reducing or inhibiting gingivitis,
f. reducing levels of acid producing bacteria,
g. increasing relative levels of arginolytic bacteria,
h. inhibiting microbial biofilm formation in the oral cavity,
i. raising and/or maintaining plaque pH at levels of at least pH 5.5 following sugar challenge,
j. reducing plaque accumulation,
k. reducing erosion of the teeth, and/or
l. cleaning the teeth and oral cavity.

## Patentansprüche

1. Mundpflegezusammensetzung in der Form eines Zahnreinigungsmittels oder einer Mundspülung, wobei die Zusammensetzung umfasst:
a. eine wirksame Menge einer basischen Aminosäure, in freier oder Salzform, wobei die basische Aminosäure Arginin ist;
b. eine wirksame Menge eines wasserlöslichen Strontiumsalzes, wobei das wasserlösliche Strontiumsalz aus Strontiumacetat; Strontiumchlorid; Strontiumnitrat; Strontiumlactat; Strontiumbromid; und einer Kombination von zwei oder mehreren davon ausgewählt ist; wobei das wasserlösliche Strontiumsalz in einer Menge von 0.1 bis 15% bezogen auf das Gewicht vorliegt,
wobei die Zusammensetzung weiterhin eine Fluoridionenquelle umfasst.

2. Mundpflegezusammensetzung nach Anspruch 1, wobei die Fluoridionenquelle aus Natriummonofluorphosphat; Zinnfluorid; Natriumfluorid; Kaliumfluorid; Natriumfluorsilikat; Ammoniumfluorsilikat; Aminfluorid; Ammoniumfluorid; und einer Kombination von zwei oder mehreren davon ausgewählt ist.

3. Mundpflegezusammensetzung nach Anspruch 1 oder Anspruch 2, die weiterhin eine Kaliumionenquelle umfasst.

4. Mundpflegezusammensetzung nach Anspruch 3, wobei die Kaliumionenquelle aus Kaliumnitrat und Kaliumchlorid ausgewählt ist.

5. Mundpflegezusammensetzung nach einem beliebigen voranstehenden Anspruch, die weiterhin einen Strontiumglas-Komplex umfasst.

6. Mundpflegezusammensetzung nach Anspruch 5, wobei der Strontiumglas-Komplex Strontium-Natrium-Phosphosilikat umfasst.

7. Mundpflegezusammensetzung nach einem beliebigen der Ansprüche 1 bis 6, zur Verwendung in einem Verfahren zum
a. Verringern oder Inhibieren der Bildung von Zahnkaries,
b. Verringern, Reparieren oder Inhibieren von frühen Zahnschmelzläsionen,
c. Verringern oder Inhibieren von Demineralisierung und Fördern von Remineralisierung der Zähne,
d. Verringern von Hypersensibilität der Zähne,
e. Verringern oder Inhibieren von Gingivitis,
f. Verringern von Spiegeln an säurebildenden Bakterien,
g. Erhöhen von relativen Spiegeln von arginolytischen Bakterien,
h. Inhibieren von mikrobieller Biofilmbildung in der Mundhöhle,
i. Erhöhen und/oder Halten von Plaque-pH auf Höhen von mindestens pH 5,5 nach einer Zucker-Challenge,
j. Verringern von Plaque-Ansammlung,
k. Verringern von Erosion der Zähne, und/oder
l. Reinigen der Zähne und Mundhöhle.

## Revendications

1. Composition de soin buccal sous la forme d'un dentifrice ou d'un rince-bouche, la composition comprenant
a. une quantité efficace d'un acide aminé basique, sous forme libre ou sous forme de sel, dans laquelle l'acide aminé basique est l'arginine ;
b. une quantité efficace d'un sel de strontium soluble dans l'eau, dans laquelle le sel de strontium soluble dans l'eau est choisi parmi l'acétate de strontium ; le chlorure de strontium ; le nitrate de strontium ; le lactate de strontium ; le bromure de strontium ; et une combinaison de deux ou plus de ceux-ci ; dans laquelle le sel de strontium soluble dans l'eau est présent en une quantité de 0,1 à 15 % en poids,
dans laquelle la composition comprend en outre une source d'ions fluorure.

2. Composition de soin buccal selon la revendication 1, dans laquelle la source d'ions fluorure est choisie parmi le monofluorophosphate de sodium ; le fluorure stanneux ; le fluorure de sodium ; le fluorure de potassium ; le fluorosilicate de sodium ; le fluorosilicate d'ammonium ; un fluorure d'amine ; le fluorure d'ammonium ; et une combinaison de deux ou plus de ceux-ci.

3. Composition de soin buccal selon la revendication 1 ou la revendication 2, comprenant en outre une source d'ions potassium.

4. Composition de soin buccal selon la revendication 3, dans laquelle ladite source d'ions potassium est choisie parmi le nitrate de potassium et le chlorure de potassium.

5. Composition de soin buccal selon l'une quelconque des revendications précédentes, comprenant en outre un complexe de strontium-verre.

6. Composition de soin buccal selon la revendication 5, dans laquelle le complexe de strontium-verre comprend du phosphosilicate de sodium et de strontium.

7. Composition de soin buccal selon l'une quelconque des revendications 1 à 6, pour une utilisation dans une méthode pour
a. réduire ou inhiber la formation de la carie dentaire,
b. réduire, réparer ou inhiber les lésions précoces de l'émail,
c. réduire ou inhiber la déminéralisation et favoriser la reminéralisation des dents,
d. réduire l'hypersensibilité des dents,
e. réduire ou inhiber la gingivite,
f. réduire les niveaux de bactéries productrices d'acide,
g. augmenter les niveaux relatifs de bactéries arginolytiques,
h. inhiber la formation de biofilm microbien dans la cavité buccale,
i. augmenter et/ou maintenir le pH de la plaque à des niveaux d'au moins pH 5,5 après épreuve par du sucre,
j. réduire l'accumulation de plaque,
k. réduire l'érosion des dents, et/ou
l. nettoyer les dents et la cavité buccale.
